# EUROPEAN PATENT APPLICATION

(11) **EP 4 582 110 A1**
(43) Date of publication of application: **09.07.2025**
(21) Application number: 23860736.0
(22) Date of filing: 16.08.2023
(51) Int. Cl.: A61L 27/20, A61L 27/54

(54) **COMPOSITION FOR HARD TISSUE REGENERATION**

(30) Priority: 01.09.2022 KR 20220110794
(71) Applicant: CG Bio Co., Ltd., Seoul 04349 (KR)
(72) Inventor: SONG, Seok Beom, Seongnam-si, Gyeonggi-do 13186 (KR); KIM, Pil Yun, Incheon 21965 (KR); HONG, Eun Pyo, Hwaseong-si, Gyeonggi-do 18392 (KR); RYU, Mi Young, Seongnam-si, Gyeonggi-do 13518 (KR)
(74) Representative: Icosa
(86) International application number: PCT/KR2023/012069
(87) International publication number: WO 2024/049058

(57) **Abstract**

Provided is a composition for hard tissue regeneration.

## Description

### [Technical Field]

The present disclosure relates to a composition for hard tissue regeneration.

### [Background Art]

All materials that are to be grafted into the human body, particularly materials for the regeneration of hard tissues such as osseous tissue or cartilaginous tissue, should have good processability and moldability or have good in-situ polymerization properties so as to be suited for wounds. These materials are required to provide a suitable environment for the adhesion, growth, and differentiation of cells, and additional products generated by degradation of the materials are also required to have biocompatibility. In particular, when the compressive strength and yield value of a graft material for bone regeneration are too low, it is difficult to maintain the abilities of the graft material to fix its location and to maintain its external shape in the closure or implant placement stage after injection or dense filling of the graft material. In addition, if the adhesiveness of a graft material is too high, it can easily stick to a surgical tool during surgery, and thus it is difficult to easily fill bone defects, resulting in a decrease in workability.

In the initial stages of development, such graft biomaterials for hard tissue regeneration relied on their characteristic of being inert *in vivo,* but the use thereof was significantly limited due to infection and inflammatory reactions which occur in the surrounding tissue after implantation. Since then, with the rapid development of biomaterial technologies based on metals, ceramics, and polymers, materials that are biocompatible rather than bioinert were designed and developed, leading to the development of bioactive scaffolds for bone tissue regeneration, which vary depending on the site and purpose of use. It is required that such bioactive scaffolds for bone tissue regeneration have different physical properties depending on the location of graft placement; are not toxic to the surrounding tissue; and have relatively high mechanical properties compared to those for other artificial organs. Such bioactive scaffolds for bone tissue regeneration have been marketed and developed as various biomaterials depending on the properties of the raw materials and the intended use thereof.

On the other hand, in the case of hard tissue, the shape of the damaged area is often irregular due to the nature of the tissue, making it difficult to select a graft material that is suitable for the defect area. Accordingly, it is necessary to find a material that has adequate strength and excellent adhesiveness to surrounding tissues, is easily injectable to fill irregular damaged areas, and above all, can promote tissue regeneration.

### [Disclosure]

### [Technical Problem]

An object of the present disclosure is to provide a composition for hard tissue regeneration, comprising a crosslinked hyaluronic acid and a transforming growth factor-beta (TGF-β), wherein the crosslinked hyaluronic acid is a particle having an average diameter of 3 µm to 250 µm.

Another object of the present disclosure is to provide a kit for hard tissue regeneration, comprising the composition for hard tissue regeneration, a mixing tool, and an injection tool.

### [Advantageous Effects]

A composition of the present disclosure exhibits high adhesive force and viscosity and a low extrusion force, and thus has physical properties suitable as a grafting material for hard tissue regeneration. Therefore, the composition may be easily injected into a defect area of tissue and may also maintain its shape during the regeneration period of the corresponding tissue. Accordingly, excellent clinical effects on hard tissue regeneration may be expected.

### [Brief Description of the Drawing]

FIG. 1 shows adhesive force according to a particle size of hyaluronic acid in a composition of the present disclosure;
FIG. 2 shows viscosity according to a particle size of hyaluronic acid in the composition of the present disclosure;
FIG. 3 shows adhesive force according to a mixing ratio of hyaluronic acid and a buffer in the composition of the present disclosure;
FIG. 4 shows viscosity according to a mixing ratio of hyaluronic acid and a buffer in the composition of the present disclosure;
FIG. 5 shows extrusion force according to a mixing ratio of hyaluronic acid and a buffer in the composition of the present disclosure;
FIG. 6 shows preparation of a microfracture animal model with a defect site on a trochlear groove using a rabbit and a tissue regeneration method using the same;
FIG. 7 shows the scoring criteria of the OARSI assessment system;
FIG. 8 shows the course of the OARSI score according to the administration of the composition for hard tissue regeneration in a microfracture animal model according to one embodiment of the present disclosure;
FIG. 9 shows the scoring criteria of the subchondral bone grading system; and
FIG. 10 shows the course of the subchondral bone grade according to the administration of the composition for hard tissue regeneration in a microfracture animal model according to one embodiment of the present disclosure.

### [Detailed Description of Preferred Embodiments]

Each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific descriptions below.

Further, those skilled in the art will recognize, or be able to ascertain using no more than routine experimentation, many equivalents to the specific embodiments of the disclosure described herein. Further, these equivalents should be interpreted to fall within the present disclosure.

Furthermore, throughout the specification of the present disclosure, when a part is referred to as "comprising" an element, it will be understood that other elements may also be included rather than other elements being excluded unless otherwise specially described.

Hereinafter, the present disclosure will be described in more detail.

To achieve the above objectives, a first aspect of the present disclosure provides a composition for hard tissue regeneration, comprising a crosslinked hyaluronic acid and a transforming growth factor-beta (TGF-β).

In this regard, the crosslinked hyaluronic acid may be characterized in that it is a particle having an average diameter of 3 µm to 250 µm.

For example, the tissue that may be regenerated by applying the composition of the present disclosure may be cartilaginous tissue or osseous tissue, but is not limited thereto.

As used herein, the term "hyaluronic acid (HA)", also called hyaluronan, is an anionic, non-sulfated glycosaminoglycan distributed widely throughout connective, epithelial, and neural tissues. Hyaluronic acid is unique among glycosaminoglycans because it has a non-sulfated form, is formed in the plasma membrane instead of the Golgi apparatus, and may be very large. The average 70 kg (150 lb) person has roughly 15 g of hyaluronan in the body, one third of which is degraded and synthesized every day. As one of the chief components of the extracellular matrix, hyaluronic acid significantly contributes to cell proliferation and migration, and is involved in the progression of many malignant tumors.

As used herein, the term "crosslinked hyaluronic acid" is classified as a category distinct from the linear hyaluronic acid described above, and may be distinguished according to the type of processing and the composition of the acid. The crosslink refers to a process of obtaining a new structural material composed of a plurality of hyaluronic acid filaments having a higher molecular weight by linking the linear hyaluronic acid molecules together. High-molecular-weight hyaluronic acid formed through crosslinking may lose the properties of individual molecules to reach a state like a real gel. The linear hyaluronic acid described above is a substance which is first used and commercialized, and is composed of unchanged linear hyaluronic acid chains, whereas crosslinked hyaluronic acid is a substance which is more recently developed, and is characterized by comprising crosslinks formed between several linear hyaluronic acid molecules at the production stage. These crosslinks are transversal bridges that join a plurality of chains together for the purpose of forming hyaluronic acid macromolecules having a larger size, a higher molecular weight, and consequently, a higher density and durability.

For example, the crosslinked hyaluronic acid may be crosslinked by one or more crosslinking agents selected from the group consisting of 1,4-butanediol diglycidyl ether (BDDE), divinyl sulfone (DVS), bis ethyl carbodiimide (BCDI), polyethylene glycol (PEG), and 1-ethyl-3-(3-dimethylaminopropyl) carbodiimide (EDC).

For example, the composition may comprise crosslinked hyaluronic acid in an amount of 15 mg/mL to 30 mg/mL, based on the volume of the final composition.

As used herein, the term "transforming growth factor-beta (TGF-β)" is secreted by many cell types, comprising macrophages, in a latent form in which it is complexed with two different polypeptides, latent TGF-beta binding protein (LTBP) and latency-associated peptide (LAP). One of its key functions is the regulation of inflammatory processes, particularly, in the gut. TGF-β also plays a crucial role in stem cell differentiation as well as T-cell regulation and differentiation. Because of its role in immune and stem cell regulation and differentiation, it is a highly researched cytokine in the fields of cancer, autoimmune diseases, and infectious diseases. The TGF-β superfamily comprises endogenous growth inhibiting proteins. An increase in TGF-β expression often correlates with the malignancy of many cancers and a defect in the cellular growth inhibition response to TGF-β. Its immunosuppressive functions come to dominate, contributing to oncogenesis. The dysregulation of its immunosuppressive functions is also implicated in the pathogenesis of autoimmune diseases, although their effect is mediated by the environment of other cytokines present.

For example, the TGF-β comprises, without limitation, proteins belonging to the TGF-β superfamily, which is a large group of structurally related cell regulatory proteins that interact with TGF-β receptors. Specifically, the TGF-β may be one or more selected from the group consisting of TGF-β1, TGF-β2, TGF-β3, BMP-2, BMP-4, BMP-5, BMP-6, and BMP-7. More specifically, the TGF-β may be TGF-β3, and the TGF-β3 may be, but is not limited to, human gene recombinant TGF-β3 derived from E. *coli.*

As used herein, the term "TGF-β3" is a protein encoded in humans by the TGFB3 gene. It is a type of protein, known as a cytokine, which is involved in cell differentiation, embryogenesis, and development. It belongs to a large family of cytokines called the transforming growth factor-beta superfamily, which comprises the TGF-β family, bone morphogenetic proteins (BMPs), growth and differentiation factors (GDFs), inhibins, and activins.

For example, the composition may comprise the TGF-β, e.g., TGF-β3 in an amount of 0.003 mg/mL to 1.0 mg/mL, based on the volume of the final composition. For example, the composition may comprise the TGF-β in an amount of 0.005 mg/mL to 1.0 mg/mL, 0.006 mg/mL to 1.0 mg/mL, 0.005 mg/mL to 0.5 mg/mL, 0.005 mg/mL to 0.3 mg/mL, or 0.006 mg/mL to 0.2 mg/mL, based on the volume of the final composition, but is not limited thereto. When TGF-β is comprised in an amount less than the above amount, it may be difficult to achieve a desired level of a synergistic effect according to TGF-β addition, and even though TGF-β is comprised in an amount exceeding the above amount, it may be difficult to expect an additional effect according to the added amount.

For example, the composition may have an adhesive force of 2 N to 15 N. For example, the composition may have an adhesive force of 3 N to 12 N, or 4 N to 10 N, but is not limited thereto.

Further, the composition may have a viscosity of 8,000 cP to 80,000 cP. For example, the composition may have a viscosity of 10,000 cP to 60,000 cP, 15,000 cP to 50,000 cP, or 20,000 cP to 40,000 cP, but is not limited thereto.

Furthermore, the composition may have an extrusion force of 3 N to 50 N. For example, the composition may have an extrusion force of 3 N to 50 N, 10 N to 50 N, 10 N to 45 N, 15 N to 45 N, or 15 N to 35 N, but is not limited thereto.

Due to the above physical properties, the composition of the present disclosure may be easily injected into damaged hard tissue, and even when injected in an environment existing in body fluids such as bone marrow, etc., it may have excellent adhesive force to the corresponding tissue, thereby maintaining its shape at the injected site.

For example, the composition may reduce the Osteoarthritis Research Society International (OARSI) score to 2.0 or less at 12 weeks after injection into a hard tissue defect site.

Further, the composition may reduce the subchondral bone grade to 1.0 or less at 8 weeks after injection into a hard tissue defect site.

As used herein, the term "Osteoarthritis Research Society International (OARSI) score" is a score formulated by OARSI in 1998 to standardize the assessment of osteoarthritis (OA) histopathology, according to safranin O/fast green staining results. The OARSI score represents a combined assessment based on the severity and extent of OA in the articular cartilage, while the total score ranges from 0 to 6. The specific standards were consistent with the previous report, and a higher OARSI score indicates more severe cartilage injury in joints.

As used herein, the term "subchondral bone grading" is an assessment system that grades the stage of subchondral bone on a scale of 0 to 3, based on previous studies describing the changes in subchondral bone and the rough observations of osteoarthritis sample material by two researchers, OMA and PL. Specifically, subchondral bone grading accounts for structural changes in the bone connected to the cartilage, and as the grade increases, it indicates that the bone becomes harder and thicker. The subchondral bone is located between the cartilage and the bone marrow, and when the subchondral bone becomes thicker, the supply of active factors for maintaining the cartilage structure from the bone marrow is blocked, which may accelerate cartilage damage.

As described above, the composition of the present disclosure reduced the OARIS score to a very low level of 2.0 or less at 12 weeks after being injected into a hard tissue defect site, and/or reduced the subchondral bone grade to a very low level of 1.0 or less at 8 weeks, which means that the defect site was regenerated to a level similar to the cartilage and/or subchondral bone before the defect.

For example, the composition may be used by mixing crosslinked hyaluronic acid and TGF-β, immediately before being injected into the hard tissue defect site.

A second aspect of the present disclosure provides a kit for hard tissue regeneration, comprising the composition for hard tissue regeneration of the first aspect, a mixing tool, and an injection tool.

For example, the mixing tool may be a mixing syringe, a vial transport device, or a connector, and the injection tool may be a syringe or a syringe needle, but is not limited thereto.

For example, the kit of the present disclosure may inject the composition into a hard tissue defect site using a syringe needle, or directly inject the composition into an open site using a syringe, etc. after a surgical procedure as an auxiliary tool.

Specifically, a vial comprising TGF-β and an empty syringe are connected to a vial transport device, and TGF-β is transported to the empty syringe. The syringe is then separated and connected to a syringe filled with crosslinked hyaluronic acid using a connector to mix the TGF-β with the crosslinked hyaluronic acid. A syringe needle is connected to the mixed syringe, and it may be directly injected into a hard tissue defect site or injected into a hard tissue defect site after a surgical operation. When used in conjunction with a surgical operation, the kit may be provided by applying a double sterilization packaging, etc. in order to minimize the possibility of infection that may occur during handling, but there is no difference in the method of use or principle.

### [Mode for Carrying Out the Invention]

Hereinafter, the present disclosure will be described in more detail with reference to exemplary embodiments. However, these exemplary embodiments are only for illustrating the present disclosure, and the scope of the present disclosure is not intended to be limited by these exemplary embodiments.

### Preparation Example 1: Preparation of Crosslinked Hyaluronic Acid

Crosslinked hyaluronic acid was prepared by reacting hyaluronic acid (molecular weight of 500,000 Da to 1,500,000 Da) and 1,4-butanediol diglycidyl ether (BDDE) as a crosslinking agent. In detail, 1 g/g of hyaluronic acid, 0.8 g/g of NaOH, 3.2 g/g of purified water, and 0.02 g/g of BDDE were mixed and stirred to form a gel. After completing the synthesis, the gel was crosslinked in a shaking incubator (shaking speed: 80 rpm, temperature: 30°C, reaction time: 19 hours). The crosslinked gel was coarsely crushed into a size of 15 mm × 10 mm and then dialyzed. The dialyzed gel was sieved through a standard sieve to obtain regular-sized particles.

### Example 1: Evaluation of Physical Properties of Crosslinked Hyaluronic Acid according to Size

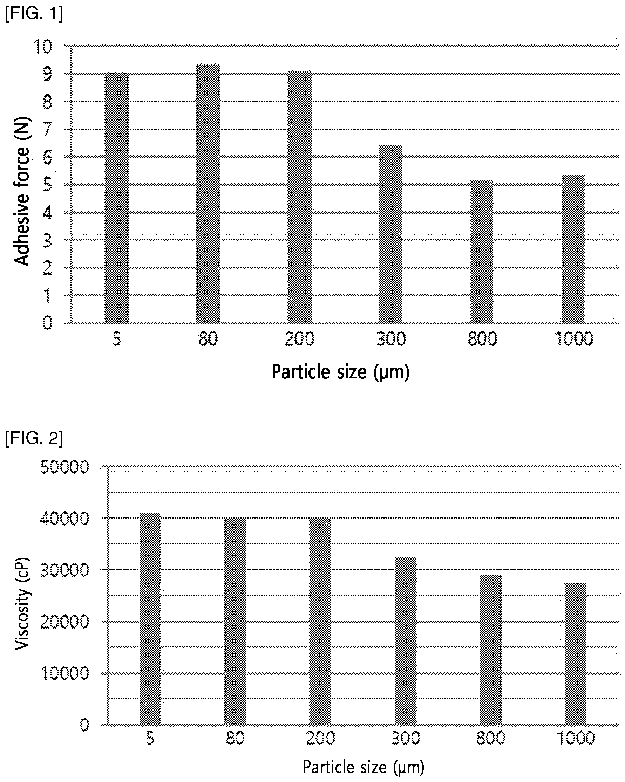

The crosslinked hyaluronic acid prepared according to Preparation Example 1 was classified into six groups according to particle size, thereby preparing samples. Specifically, samples comprising particles with an average particle size of 5 µm, 80 µm, 200 µm, 300 µm, 800 µm, or 1000 µm at a concentration of 20 mg/mL in a PBS buffer were prepared, and the adhesive force and viscosity were measured, and the results are shown in FIGS. 1 and 2, respectively. In detail, the adhesive force was measured using an Antopa rheometer at a speed of 200 µm/sec, after the sample was injected into a plate, and the spindles were lowered to a position of 0.25 mm so that they touched the sample. The viscosity was measured using a Brookfield viscometer at a speed of 0.5 rpm, after putting 0.5 cc of the sample into a sample cup.

As shown in FIGS. 1 and 2, the samples comprising crosslinked hyaluronic acid particles with an average size of 5 µm to 200 µm exhibited high adhesive force and viscosity, as compared to samples comprising particles with a larger size.

### Example 2: Evaluation of Physical Properties of Composition for Hard Tissue Regeneration Comprising Crosslinked Hyaluronic Acid and TGF-β3 according to Mixing Ratio

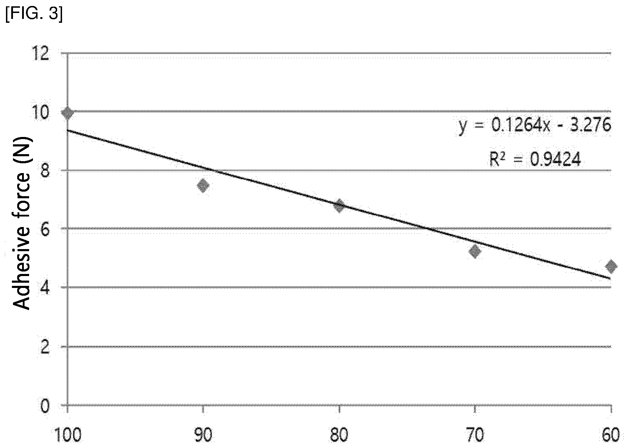

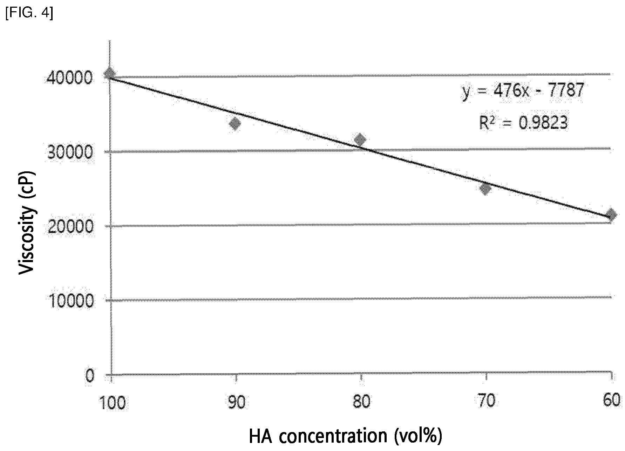

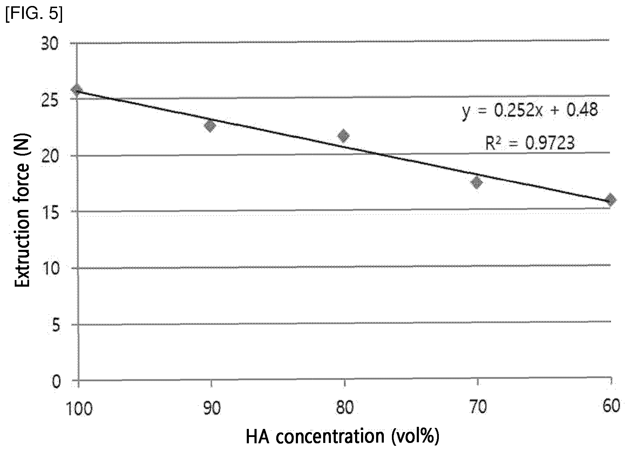

The crosslinked hyaluronic acid prepared according to Preparation Example 1 was used to prepare a 20 mg/mL solution with PBS buffer, and a series of compositions were prepared by mixing the solution with recombinant human transforming growth factor-beta3 (rhTGF-β3) at a predetermined volume ratio while changing the ratio from 10:0 to 6:4. The adhesive force, viscosity, and extrusion force of the series of compositions were measured, and the results are shown in FIGS. 3 to 5, respectively. In detail, the adhesive force and viscosity were measured in the same manner as in Example 1, and the extrusion force was measured using a universal testing machine by filling 2 cc of the sample into a 3 cc syringe, connecting a 21 G syringe, fixing the filled syringe to a jig, and pressing the same at a speed of 30 *mm*/*min.*

As shown in FIGS. 3 to 5, as the ratio of crosslinked hyaluronic acid decreased, the adhesive force, viscosity, and extrusion force all showed a tendency to decrease.

### Example 3: Verification of Cartilage Regeneration Ability using Experimental Animals

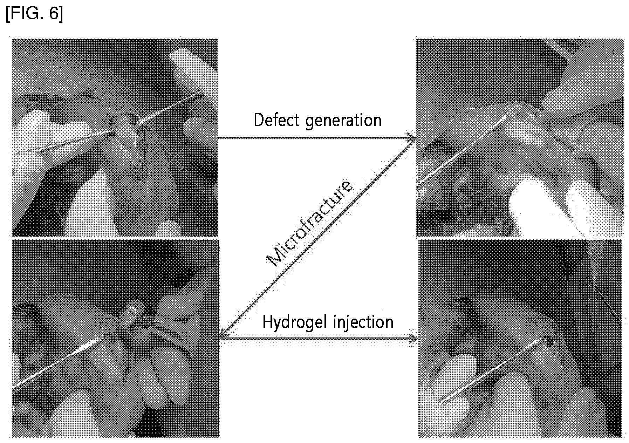

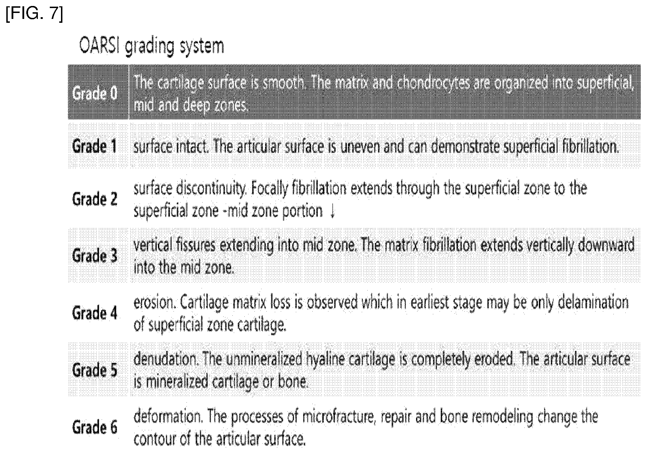

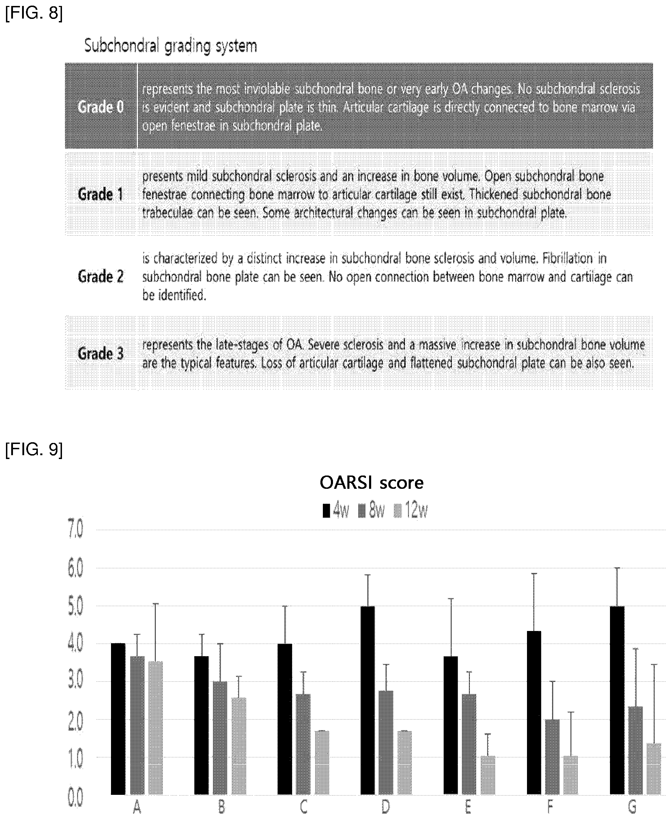

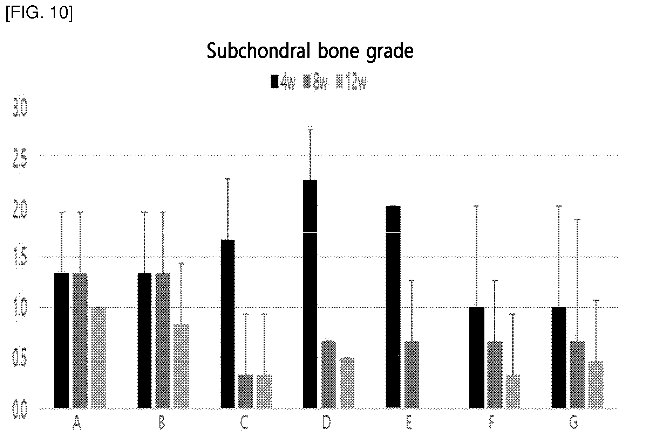

As shown in FIG. 6, a microfracture animal experiment model was created, the prepared composition was injected into the corresponding defect site, and the degree of regeneration was examined through observation for 4 weeks, 8 weeks, and 12 weeks. As control groups, an untreated group (A) and a group treated only with a carrier, *i.e.,* crosslinked hyaluronic acid (B), were used. As experimental groups, groups each injected with the composition comprising 20 mg/mL of crosslinked hyaluronic acid and TGF-β3 at a final concentration of 6 ppm, 30 ppm, 60 ppm, 100 ppm, or 200 ppm (C to G, respectively) were used. The OARSI score and subchondral bone grade were determined by scoring based on the criteria disclosed in FIGS. 7 and 8 using hematoxylin & eosin staining (H&E staining) and safranin O-fast green staining (SO-FG staining), and the results are shown in FIGS. 9 and 10, respectively. Furthermore, these values are comprehensively summarized in Table 1 below.

**[Table 1]**

| Section | Test material | OARSI score | | | Subchondral bone grade | | |
|---|---|---|---|---|---|---|---|
| | | 4 weeks | 8 weeks | 12 weeks | 4 weeks | 8 weeks | 12 weeks |
| Control group (A) | Untreated group; microfracture | 4.0±0.0 | 3.7±0.6 | 3.5±1.5 | 1.3±0.6 | 1.3±0.6 | 1.0±0.0 |
| Control group (B) | **Carrie alone;** microfracture + crosslinked HA (20 mg/mL) | 3.7±0.6 | 3.0±1.0 | 2.6±0.6 | 1.3±0.6 | 1.3±0.6 | 0.8±0.6 |
| Experimental group (C) | microfracture + crosslinked HA (20mg/mL) + TGF-β3 (6 ppm) | 4.0±1.0 | 2.7±0.6 | 1.7±0.0 | 1.7±0.6 | 0.3±0.6 | 0.3±0.6 |
| Experimental group (D) | microfracture + crosslinked HA (20mg/mL) + TGF-β3 (30 ppm) | 5.0±0.8 | 2.8±0.7 | 1.7±0.0 | 2.3±0.5 | 0.7±0.0 | 0.5±0.0 |
| Experimental group (E) | microfracture + crosslinked HA (20mg/mL) + TGF-β3 (60 ppm) | 3.7±1.5 | 2.7±0.6 | 1.0±0.6 | 2.0±0.0 | 0.7±0.6 | 0.0±0.0 |
| Experimental group (F) | microfracture + crosslinked HA (20 mg/mL) + TGF-β3 (100 ppm) | 4.3±1.5 | 2.0±1.0 | 1.0±1.2 | 1.0±1.0 | 0.7±1.2 | 0.3±0.6 |
| Experimental group (G) | microfracture + crosslinked HA (20 mg/mL) + TGF-β3 (200 ppm) | 5.0±1.0 | 2.3±1.5 | 1.4±2.0 | 1.0±1.0 | 0.7±0.6 | 0.5±0.6 |

As shown in FIGS. 9 and 10 and Table 1, at 4 weeks after injection of the composition, some experimental groups showed inferior values, as compared to the control groups. However, as the compositions hardened to 8 weeks and 12 weeks, each experimental group showed significantly improved values, as compared to the control groups, indicating that the compositions of the present disclosure exhibit superior regenerative effects on damaged cartilage after sufficient time for regeneration following the injection of the composition.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within the metes and bounds of the claims, or equivalents of such metes and bounds are therefore intended to be embraced by the claims.

## Claims

1. A composition for hard tissue regeneration, comprising a crosslinked hyaluronic acid and a transforming growth factor-beta (TGF-β),
wherein the crosslinked hyaluronic acid is a particle having an average diameter of 3 µm to 250 µm.

2. The composition of claim 1, wherein the tissue is cartilaginous tissue or osseous tissue.

3. The composition of claim 1, wherein the crosslinked hyaluronic acid is crosslinked by one or more crosslinking agents selected from the group consisting of 1,4-butanediol diglycidyl ether (BDDE), divinyl sulfone (DVS), bis ethyl carbodiimide (BCDI), polyethylene glycol (PEG), and 1-ethyl-3-(3-dimethylaminopropyl) carbodiimide (EDC).

4. The composition of claim 1, wherein the composition comprises the crosslinked hyaluronic acid in an amount of 15 mg/mL to 30 mg/mL, based on the volume of the final composition.

5. The composition of claim 1, wherein the TGF-β is one or more selected from the group consisting of TGF-β1, TGF-β2, TGF-β3, BMP-2, BMP-4, BMP-5, BMP-6, and BMP-7.

6. The composition of claim 1, wherein the composition comprises the TGF-β in an amount of 0.003 mg/mL to 1.0 mg/mL, based on the volume of the final composition.

7. The composition of claim 1, wherein the composition has an adhesive force of 2 N to 15 N.

8. The composition of claim 1, wherein the composition has a viscosity of 8,000 cP to 80,000 cP.

9. The composition of claim 1, wherein the composition has an extrusion force of 3 N to 50 N.

10. The composition of claim 1, wherein the composition reduces the OARSI score to 2.0 or less at 12 weeks after injection into a hard tissue defect site.

11. The composition of claim 1, wherein the composition reduces the subchondral bone grade to 1.0 or less at 8 weeks after injection into a hard tissue defect site.

12. The composition of claim 1, wherein the crosslinked hyaluronic acid and TGF-β are mixed, immediately before injection into a hard tissue defect site.

13. A kit for hard tissue regeneration, comprising the composition for hard tissue regeneration of claim 1, a mixing tool, and an injection tool.

14. The kit of claim 13, wherein the mixing tool is a mixing syringe, a vial transport device, or a connector.

15. The kit of claim 13, wherein the injection tool is a syringe or a syringe needle.
